# EUROPEAN PATENT SPECIFICATION

(11) **EP 1 919 932 B1**
(45) Date of publication and mention of the grant of the patent: **04.01.2012**
(21) Application number: 06792982.8
(22) Date of filing: 24.08.2006
(51) Int. Cl.: C07K 1/14, C07K 14/775

(54) **PRION INACTIVATION**
PRIONINAKTIVIERUNG
PROCEDE D'INACTIVATION DES PRIONS

(30) Priority: 25.08.2005 EP 05107818; 30.08.2005 US 712104 P
(43) Date of publication of application: 14.05.2008
(73) Proprietor: Riesner, Detlef, 40547 Düsseldorf (DE)
(72) Inventor: MÜLLER, Henrik, 99752 Kehmstedt (DE); RIESNER, Detlev, 40547, DÜSSELDORF (DE)
(74) Representative: von Kreisler Selting Werner
(86) International application number: PCT/EP2006/065639
(87) International publication number: WO 2007/023178

(56) References cited:
- US-A- 5 633 349
- US-A1- 2003 004 312
- TH. R. APPEL ET AL: "Heat Stability of Prion Rods and Recombinant Prion protein in Water, Lipid and Lipid-Water Mixtures" JOURNAL OF GENERAL VIROLOGY, vol. 82, 2001, pages 465-473, XP002364622 cited in the application

## Description

Subject of the invention is a process for the inactivation of prions in a sample which comprises prion proteins or is suspected of comprising prion proteins, the process comprising a heat treatment in the presence of a lipophilic substance comprising at least 8 carbon atoms, wherein the heat treatment is performed at a temperature below 100 °C at which the lipophilic substance is in a liquid state and under conditions wherein degradation of less than 90% of the prion proteins occurs.

Prions are the causative agents of fatal neurodegenerative diseases, among them Creutzfeldt-Jakob disease (CJD) of man, bovine spongiform encephalopathy (BSE) and scrapie of sheep. They are defined as proteinaceous infectious particles, and so far the prion protein (PrP) is the only component that correlates unequivocally with infectivity. In the infected organism, PrP is present both in the cellular form, PrP^{C} as well as in an abnormal, scrapie isoform, PrP^{Sc}. Upon purification using detergents and digestion with proteinase K, PrP^{Sc} is transformed into an N-terminally truncated but still infectious form of 27-30 kDa designated PrP 27-30. PrP^{Sc} and the truncated PrP 27-30 form large and insoluble aggregates. These are visible in the infected brain in a wide variety of sizes and shapes, i.e. from rather diffuse depositions to solid plaques or amyloidic fibrillar forms. The latter are called also prion rods or scrapie-associated fibrils. The aggregates result from lipophilic interactions, and the prion protein has been described as a lipophilic protein (Prusiner *et al.,* 1981). Therefore, PrP is assumed to have a strong tendency for lipophilic interactions and to bind lipids.

Prion proteins are characterized by an unusual resistance to the thermal or chemical treatments which are commonly used to inactivate agents of infectious diseases (Taylor, 2004). The stability and the tendency to form lipophilic aggregates appear closely connected. The extraordinary stability of prions to physical and chemical inactivation is considered today as the major cause of the BSE epidemic resulting from feeding of insufficiently inactivated meat-and-bone meal to cattle. Besides, a variant of CJD is most probably caused by the consumption of infected cattle products. However, also less obvious routes of human exposure to infectious material might exist. Bovine tallow and bone fat are widely used as raw materials for oleochemical processes, soap and detergents, cosmetics and animal feed.

There is a strong need for methods by which prions are inactivated. Almost all technical processes applied to bovine fat involve heating, usually in the presence of water. The effect of such treatments on prion proteins was studied in Appel *et al.,* 2001 (II). They found that the presence of lipids increases the heat stability of the prion rods. The authors conclude that in the presence of lipids a temperature above 170° C is necessary in order to obtain sufficient degradation of the prions.

The effects of hydrolytic fat splitting under pressure are studied in Appel *et al.,* 2001 (I). In this process, temperatures exceeding 200° C are applied for at least 20 min in water-saturated atmosphere under pressure. The authors studied the prion degradation at different temperatures during this process. The authors conclude that in an autoclaving process, a sufficient prion inactivation with a factor > 10⁴ is obtained at a temperature of 145 °C or 195 °C.

According to another method of US 6,720,355, the prions are inactivated by a treatment at elevated temperatures in the presence of sodium dodecyl sulfate (SDS).

In another method of the state of the art disclosed in US 6,719,988, the prions are inactivated at elevated temperature in the presence of alkyl sulfates at an acidic pH ranging from 2.5 to 4.5.

In the method of US 6,743,899, the prions are inactivated in a basic solution with a pH between 10 and 13.

US-A-2003/0004312 discloses an antiseptic composition useful in destroying the infectivity of infectious proteins such as prions. The antiseptic composition is preferably maintained at either a low pH of 4.0 or less or a high pH of 10.0 or more either of which allows for an environment under which the active component (which is preferably sodium dodecyl sulfate) destroys infectivity. The composition may be added to blood, blood products, collagen, tissues and organs prior to transplantation. The composition also may be added to livestock feed to denature any prions in the livestock. Methods of denaturing infectious proteins are also disclosed which method can use but do not require higher temperatures and long period of exposure.

However, the known methods for inactivating prions require relatively drastic conditions under which degradation of the prion proteins occurs, i.e. high pressure, highly acidic or basic conditions, the addition of aggressive substances like alkyl sulfates and/or high temperatures.

The problem underlying the present invention is to provide a new process for inactivating prions under mild conditions. The process should be applicable to a wide range of applications, for example for the cleaning of medical instruments or instruments used in pharmaceutical or food industry. The process should also be applicable for the treatment of compositions comprising fat from animal samples, such as tallow. The process shall be handled easily and shall be widely applicable in an industrial scale.

Surprisingly, the problem underlying the present invention is solved by a process of any of claims 1-20.

Subject of the invention is a process for the inactivation of prions in a sample which comprises prion proteins or is suspected of comprising prion proteins. The process comprises a heat treatment in the presence of a lipophilic substance, which comprises at least 8 carbon atoms. The heat treatment is performed at a temperature below 100 °C, at which the lipophilic substance is in a liquid state and under conditions wherein the prion proteins are not completely degraded.

"Degradation" of prion proteins means, that the peptide backbone of the prion proteins is degraded.

"Inactivation" as used herein means, that the prion proteins lost their capability to induce the neurodegenerative diseases such as scrapie, which they are known to cause in their active form. A prion protein is not necessarily degraded to be inactivated. By the method of the invention, the vast majority of the prion proteins is inactivated (e. g. by a factor of 10⁵), whereas in particular only up to 99 % of the prion proteins are degraded. In a less stringent embodiment only up to 95 % in particular 90 % of the prion proteins are degraded.

"Lipophilic substance" means that the substance dissolves more easily in lipid than in water. Preferably, the lipophilic substance is selected from the group consisting of fats, fatty acids, cholesterol, oils, fatty acid derivatives, tallow and mixtures thereof. In preferred embodiments, the lipophilic substance comprises at least 10, 12 or 14 C-atoms. In another preferred embodiment, the lipophilic substance is not an aggressive substance. It is preferred that the lipophilic substance is not an amphiphilic substance with a hydrophilic moiety which is a strong acid or base or the salt of a strong acid or base, such as sodium-SDS. Preferably, the pKₛ of an acid or basic moiety of the lipophilic substance of the invention is < 3, more preferably < 1; the pK_{b} is preferably > 11, more preferably > 13.

The process of the invention is performed under mild conditions. In contrast to the known processes of the state of the art, the prion proteins are not entirely degraded, i.e. hydrolyzed but nonetheless mostly inactivated. Preferably the inactivation rate is at least 10⁴ or 10⁵, more preferably 10⁶ or 10⁷.

It is a completely unexpected and unprecedented result that prion proteins can be inactivated under such mild conditions in the presence of lipophilic substances such as fats. In contrast, according to the state of the art it was assumed that the presence of lipophilic substances such as lipids, tallow, fatty acids and glycerol would stabilize the prions against heat inactivation. Therefore, Appel *et al.* (2001, I and II) teach that in order to obtain an efficient inactivation of prions in the presence of lipids, the temperature has to be relatively high, preferably at least 170° C. The authors of Appel *et al.* I, II did not realize that a prion inactivation would be efficient at relatively low temperatures, because in accordance with the common knowledge they only looked at prion degradation. They found, that protein degradation at a temperature of 100 °C and also at higher temperatures was not efficient, because the degradation levels were far below required levels of approximately 10⁵ or more.

Surprisingly, according to the present invention the prion inactivation is effective under mild conditions at a temperature below 100° C. At temperatures of 80° C, an inactivation factor of about 10⁶ is obtainable with a mixture of 90 % tallow and 10 % water. In contrast, at 80° C an inactivation factor of only about 10 is obtained for water and even less for glycerol. Unexpectedly, at low temperatures and under mild conditions the lipophilic substances obviously do not stabilize the prion proteins, as suggested by the prior art, but seem to destabilize them strongly. High levels of inactivation are observed, even though degradation of prion proteins under these conditions is below 90%.

The invention allows a completely new method for the deactivation of prions and therefore for the prevention of diseases such as BSE and CJD. A sample which is suspected of comprising prions is treated under mild conditions, for example for 20 min at 80° C. An inactivation of prions of at least about 10⁴ is obtainable.

Preferably, the heat treatment of the invention is performed at a temperature below 95° C, more preferably below 90° C or below 80° C. Preferably, the temperature is at least 60 °C or 70 °C.

In a preferred embodiment, the process of the invention is performed at a pH above 4 and below 10, more preferably above 5 and below 9, and even more preferably between 6 and 8.

The addition of aggressive substances, such as detergents, is not necessary. In preferred embodiments, the process of the invention is performed in the absence of detergents, alkyl sulfates or SDS. The process of the invention is preferably performed without increased pressure. At a temperature below 100 °C, the method of the invention can be performed without an autoclave.

The process of the invention allows the inactivation of the prions, although the prion proteins are not completely degraded. Preferably, the process of the invention is performed under conditions such that essentially no degradation of the prion proteins occurs. In other preferred embodiments, less than 99, 95 or% of the prion proteins are degraded. The degradation rate is determined by techniques known in the art, e.g. by SDS-PAGE, Western blot and antibody detection as described in Appel *et al.* (I, II) or by bioassays.

In another preferred embodiment of the invention, the heat treatment is performed in the presence of water. Preferably, the amount of water present in the heat treatment step is between 1 and 90% (vol/vol), more preferably between 2 and 50% or 3 and 30%.

In the process of the invention, most of the inactivation of the prions occurs during the first 10 or 15 minutes of the treatment. Afterwards, the inactivation of the residual prion activity occurs at a lower rate. Therefore, in a preferred embodiment the heat treatment takes place for a time between 1 and 30 minutes, more preferably between 2 and 20 minutes, most preferably between 5 and 15 minutes. However, the process of the invention might also be applied for longer intervals, e.g. up to 1, 2 or 24 hours.

The process of the inactivation is applicable for samples which comprise prion proteins. Generally, the samples might be any liquids, materials, devices, instruments or building parts which comprise or are suspected of comprising prions. Typically, such samples comprise or have contacted animal canvas or body liquids, such as blood, meat or tallow. The inventive process is also useful for treating samples when there are legal requirements to perform prion inactivation treatments. For example, the process of the invention may be applied for the sterilization of medical devices and production devices of pharmaceutical or cosmetic products, in particular blood products. The method is also applicable for the sterilization of devices or instruments used in the processing of substances derived from animals, such as meat, fat, tallow and the like. The process of the invention is also useful for the disinfection of waste. In a building wherein potentially infectious material is handled, the floors or the walls can be treated by the process of the invention.

In a preferred embodiment, for example in the sterilization of instruments or devices, the lipophilic substance is added prior to the heat treatment step. If the sample is a substance like tallow or an animal fat, which already comprises or consists of sufficient lipophilic substances, the addition of further lipophilic substances might not be necessary, and the heat treatment may be applied directly.

In places such as hospitals, pharmaceutical production sites and slaughter houses, there is a strong need for simple and effective procedures for the inactivation of prions. The process of the present invention allows the sterilization of devices and instruments in a very easy and efficient way. The process of the invention requires comparatively low amounts of energy due to the low temperatures used. It is applied under mild conditions, such that the instruments or devices are not impaired even after repetitive sterilization. It is a great advantage of the inventive sterilization process that aggressive ingredients such as detergents, acids or bases can be avoided. The process of the invention is also advantageous, because it may be performed within short time periods, which allows to save considerable time as well as energy in industrial applications.

After a device or an instrument is treated, in a preferred embodiment the lipophilic substance and, if present, the water or additives admixed with it, are discarded. In a special embodiment of the invention, residual lipophilic substances and/or additives may be removed with an organic solvent such as methanol, ethanol or chloroform.

The process of the invention can be performed in the presence of further substances. Preferably, such additives are selected from the group consisting of chaotropic salts, oxidizing agents, reducing agents, detergents and/or aldehydes. Further chemical substances with no or limited inactivating effect on prions but which might be useful in the heat treatment of the invention are acetone, beta-propiolacetone, chlorine dioxide, diethylether, ethylene oxide, heptane, hexane, hydrogen peroxide, iodine, iodide, iodophores, potassium permanganate, sodium-dichloro-isocyanurate (NADCC), sodium periodate, peracetic acid, perchlorethylene, petrolium ether and benzene.

Chaotropic salts are those which disrupt molecular structures of proteins by adversely affecting nonbinding forces, such as Van der Waals force and hydrophobic effects. In preferred embodiments, the chaotropic salts are urea, guanidinium-hydrochloride (GdnHCl) and guanidinium isothiocyanate (GdnSCN). Chaotropic agents like urea, guanidinium hydrochloride and guanidinium isothiocyanate disrupt hydrogen bonds within the polypeptide a-helices and β-sheets of the prions. Thus, the most important structural elements of the protein molecules are destabilised. In general, the destruction leads to an irregularly coiled polypeptide chain and unrestricted mobility of the amino acid elements within the chain. Disulfide bridges (present in the PrP^{Sc} protein between amino acid residue 179 and 214) are not affected by either substance. GdnSCN is more effective than GdnHCl in terms of the destruction of hydrogen bonds. A complete conversion of prions into a irregular coiled structure of the protein can be observed in 6 N guanidinium hydrochloride (573 g/I) or in 8 N urea (480 g/l).

Guanidinium isothiocyanate has a partially denaturing effect in a 1.5 N solution (177 g/l). It inactivates prions in a concentration of 3 M after immersion times longer than 1 hour. Using a concentration of 4 M and at least 15 minutes residence time no experimental animal in various experiments is reported to become ill after intracerebral inoculation. The effect of GdnSCN is based solely on the denaturing potential and not on degradation or detaching effects. GdnSCN has an extraordinary long half-life of 1 year. Thus, it is the solution of choice as it does not have the disadvantages of sodium hydroxide (corrodes aluminium and zinc surfaces) nor the effect of sodium hypochlorite on sensitive instruments (corrodes not only aluminium and zinc but all oxidatively vulnerable metals and thus also stainless steel). The strong denaturing effect of GdnSCN can be inhibited by adding alcohol. The use of GdnSCN together with acids may lead to a release of toxic cyanide gas. After the decontamination process a crystallization of GdnSCN on surfaces of the instruments should be avoided by rinsing the instruments with water.

In preferred embodiments, the detergents are sodium dodecyl sulfate (SDS), sodium cholate, sodium deoxycholate, octylglucoside, dodecyldimethylamine oxide, 3-[(3-cholamidopropyl)dimethylammonio]-1-propanesulfonate (CHAPS), dodecyltriethylammonium bromide (DTAB), cetyltrimethylammonium bromide (CTAB), alkyl sulfate, alkyl sulfonate, polyoxyethylene-p-isooctylphenyl ether (e.g. Triton X-114, Triton X-100, Triton X-20). The detergents affect the tertiary structure of proteins, even at minute concentrations. They interact with hydrophilic and hydrophobic areas of the protein molecule and are largely responsible for loosening the hydrophobic centre of the protein molecule. Thus, complex micelles are formed.

Sodium dodecylsulfate (SDS) binds tightly to the peptide backbone and denatures proteins particularly when the interaction is enhanced by heat. SDS is used to present alkali burns on skin and mucosa and corrosion of faintly anodised aluminium or zinc surfaces. The emulsifying activity of this substance has proven particularly useful for doing so. Accordingly, the observed mechanism of decontamination appears to be a detachment and destabilisation rather than a degradation of PrP^{Sc}. The utensils are heated in an SDS solution (3% or greater; 0,10 M) for at least ten minutes. Some residual infectivity remains after the treatment with SDS unless the material is also sterilised for an hour at 121 °C. Thus in combination with autoclaving, a three-minute exposure to SDS is sufficient.

Preferred reducing agents are β-mercaptoethanol and dithiothreitol (DTT). They mainly exert an influence on the disulfide bridges. Upon cleavage of intramolecular disulfide bridges, the stability of the protein conformation is lost and irreversible denaturation is facilitated.

Preferably, the aldehydes are formaldehyde, glyoxal, succinic dialdehyde or glutaraldehyde. Formaldehyde and glyoxal can establish links between closely located segments of the peptide chains. Although the structure is stabilised by these links, it is assumed that unspecific links block contact points to other PrP molecules which are essential for an infection (Hörnlimann B, 2001).

In preferred embodiments, the alcohols are 1- and 2-propanol. Alcohols such as ethanol and 1- and 2-propanol alone do not have an effect on prions. This might be due to the stable conformation of the PrP^{Sc} and the general effect of alcohols in stabilising the structure of prions.

When using additives in the process of the invention, it is preferred that the substances are not toxic (Hörnlimann B, 2001). When using non-toxic substances, additional steps of their removal after the heat treatment can be avoided. Therefore, depending on the application of the prion inactivation process of the invention, it is mostly preferred to use mild additives. However, there might be applications wherein harsh additives such as sodium hydroxide, sodium hypochlorite might be useful.

It is especially preferred to perform the prion inactivation of the invention in the presence of a chaotropic agent, preferably urea and/or a reducing agent, preferably dithiothreitol. In preferred embodiments, the concentration of urea is 0.5 - 6 M, more preferred 1 - 3 M; and the concentration of DTT is 0.1 to 2 M, more preferred 0.25 to 1 M. Surprisingly, the prion inactivation of the invention is enhanced considerably in the presence of these additives. Subject of the invention is also a process for the inactivation of prions in a sample, which comprises prion proteins or is suspected of comprising prion proteins, the process comprising a heat treatment in the presence of a lipophilic substance comprising at least 8 carbon atoms, and further a chaotropic salt and a reducing agent, wherein the heat treatment is performed at a temperature at which the lipophilic substance is in a liquid state.

### Examples:

### Prion protein samples

Prion rods from the scrapie strain 263K were kindly obtained from Dr. S. B. Prusiner and Ana Serban (University of California, San Francisco, USA) and were prepared from Syrian Hamsters as described earlier (Prusiner *et al.,* 1983; Diringer *et al.,* 1997). Prion rods represent the most purified, concentrated, and stable form of TSE infectivity known so far, since they are formed by detergent extraction and extensive proteolysis of brains of terminally scrapie-sick Syrian golden hamsters.

### Fat

Bovine edible tallow was provided by the European Oleochemicals and Allied Products Group (APAG). The fat had been recovered from adipose tissue and bones obtained at a bovine only slaughterhouse. It was produced by dry melting at 100 °C followed by purification of the lipid fraction from tissues and proteinaceous matter by filtration through a diatomaceous earth bed. The free fatty acid content was 0.25 %. Remaining levels of moisture and total insoluble impurities were 0.11 % and 0.018 %, respectively.

### Treatment procedure

An inactivation system (Appel *et al.,* 2001) comprising a 50 ml pressure reactor, an external electric heating, temperature control, and magnetic stirrer (Parr Instrument Company reactor 4591 with Parr 4842 controller) was loaded with prion rods in a mixture of bovine edible tallow and water and heated to a temperature between 40 °C and 100 °C. Dependent upon the reactor content all temperatures were reached within 10 min. In presence of water the pressure inside the reactor was similar to the vapour pressure of water at the respective temperature. In absence of water the pressure was at or near atmospheric. The whole time the reactor content was stirred at 150 rpm to permit a fast-as-possible heating to the target temperature and an even temperature distribution within the reactor vessel. After the residence time had expired, the reactor was cooled to 40 °C by immersion into cold water. To purify for quantification the amount of prions remaining after heat treatment, a separation method being based on a methanol chloroform precipitation (Wessel and Flügge, 1984) was optimised especially for the quantitative analysis of small amounts of prions in the presence of a large excess of lipids and glycerol. A minimum PrP recovery rate of 95 % was achieved. The infectivity titre was also not decreased after methanol chloroform precipitation of prion rods subjected to 40 °C for 20 min.

### Biochemical testing for prions

The prion protein remaining undegraded after heat treatment was separated by SDS-polyacrylamide gel electrophoresis according to the protocol of Laemmli (1970). SDS-PAGE was carried out in 12 % polyacrylamide gels for 3 h. The amount of undegraded PrP was quantitatively detected by an improved immunoblot comprising staining with a mixture of the monoclonal antibodies 3F4 and R1 and enhanced chemiluminescence. Membranes were blocked with 5 % non-fat milk protein in TBST (PBS plus 0.1 % Tween 20) for 1 h at room temperature. Blocked membranes were incubated with 3F4 and R1 at 1:5000 in TBST for 1 h at room temperature. Following incubation with primary antibodies, the membranes were washed 3 x 5 min. in TBST, incubated with horseradish peroxidase-labeled secondary antibody diluted 1:5000 in TBST for 1 h at room temperature and washed again 6 x 5 min. in TBST. After chemiluminescent development for 1 min., films were exposed to Hypermax film (Amersham). The sensitivity threshold of the method was 1 ng PrP determined by dilution series of prion rods. Developed films were digitised at a resolution of 300 d.p.i., 256 grays, and saved as uncompressed TIF files. For densitometric quantification, the analysis software Scion Image 4.0.2 was used (free download from http://www.scioncorp.com) permitting to translate size and density of PrP-specific bands into a distribution function. The integrals of PrP-specific bands were quantified by comparison with at least three standards of known PrP amounts on the same gel which had also been subjected to recovery from an equal lipid-containing mixture.

### Biological testing for prions

Bioassays of prion infectivity were performed by inoculation of weanling female Syrian gold hamsters at an age of 34-38 days. After heat treatment of 15 µg prion rods and recovery from the fat water mixture, each sample was suspended in phosphate-buffered saline (PBS) pH 7.4 to a final volume of 600 µl. Samples were quick-frozen by immersion in liquid nitrogen and stored at -80 °C. For each determination of remaining infectivity, five animals were inoculated intracerebrally with 50 µl of a given specimen and examined for the development of clinical neurological disease at least twice a week. Only coded information was displayed on hamster boxes to avoid observer bias. The bioassays were terminated 390 days after inoculation. Prion titres were calculated by measuring the incubation time intervals from inoculation to onset of clinical symptoms (Prusiner *et al.,* 1982). In order to determine worst case reduction factors for the inactivation of prion infectivity the underlying dose response curve was utilised for incubation times up to 140 days. In case of incubation times longer than 140 days, the corresponding animals were not included in the calculation of the inactivation factors. Instead, the bioassay detection limit of log2 ID₅₀ was used. This is a conservative treatment to avoid an overestimation of the reduction achieved. The original data from the titration experiments, i.e. mortality rates, mean incubation times, and calculated infectivity titres are presented in **Table 1**.

Table 1 shows the effect on the 263K strain of scrapie agent of heat treatment under the conditions indicated. For calculation of mean incubation periods, infectivity titres, and standard errors only diseased animals were utilised.

**Table 1:**

| | **No. infected/** | **Mean incubation period ± SE** | **Infectivity titre ±** |
|---|---|---|---|
| **Treatment** | | | **SE** |
| | **No. injected** | **[days]** | **[log ID₅₀ per sample]** |
| **controls** (50 % tallow/ 50 % glycerol, 200 °C, 20 min.) | 0/5 | - | - |
| starting titre | 10/10 | 74 ± 1 | 7.9 ± 0.5 |
| after MeOH/CHCl₃ precipitation | 5/5 | 68 ± 3 | 8.9 ± 1.4 |
| | | | |
| | | | |
| **90 % fat/ 10 % water** 70 °C/ 20 min. | 5/5 | 94 ± 4 | 5.2 ± 0.4 |
| 80 °C/ 20 min. | 4/4¹ | 93 ± 1 | 5.3 ± 0.2 |
| 90 °C/ 20 min. | 9/9¹ | 108 ± 2 | 3.9 ± 0.2 |
| 100 °C/ 20 min. | 5/5 | 118 ± 10 | 3.2 ± 0.7 |
| 110 °C/ 20 min. | 10/10 | 124 ± 8 | 2.7 ± 0.6 |
| | | | |
| 90 °C/ 1 h | 5/5 | 104 ± 1 | 4.2 ± 0.1 |
| 90 °C/3h | 5/5 | 120±6 | 3.0±0.4 |
| 90 °C/9h | 4/4¹ | 108 ± 4 | 3.9±0.3 |
| | | | |
| 90 °C/ 20 min./ 10⁻¹ | 3/4 | 151 ± 16 | |
| 90 °C/ 20 min./ 10⁻² | 3/5 | 182 ± 23 | |
| 90 °C/ 20 min./ 10⁻³ | 0/5 | - | 4.2 ± 0.0 |
| 90 °C/ 20 min./ 10⁻⁴ | 2/5 | 195 ± 40 | |
| 90 °C/ 20 min./ 10⁻⁵ | 0/5 | - | |
| 90 °C/ 20 min./ 10⁻⁶ | 1/5 | 150 ± 0 | |
| | | | |
| **75 % fat/ 25 % water** 90 °C/ 20 min. | 5/5 | 93 ± 3 | 5.3 ± 0.4 |
| 90 °C/ 20 min./ 2.25 M Urea/ 0.5 M DTT | 5/5 | 141 ± 3 | < 2.0 |
| | | | |
| | | | |
| **50 % fat/ 50 % water** | | | |
| 90 °C/ 20 min. | 4/4¹ | 110 ± 1 | 3.8 ± 0.1 |
| | | | |
| **100 % water** 80 °C/ 20 min. | 5/5 | 78 ± 4 | 7.2 ± 0.7 |
| 90 °C/ 20 min. | 5/5 | 85 ± 1 | 6.2 ± 0.2 |
| 110 °C/ 20 min. | 5/5 I | 97 ± 1 | 5.0 ± 0.1 |
| | | | |

| | | | |
|---|---|---|---|
| ⁽¹⁾ One hamster found dead without clinical signs or PK resistance. | | | |

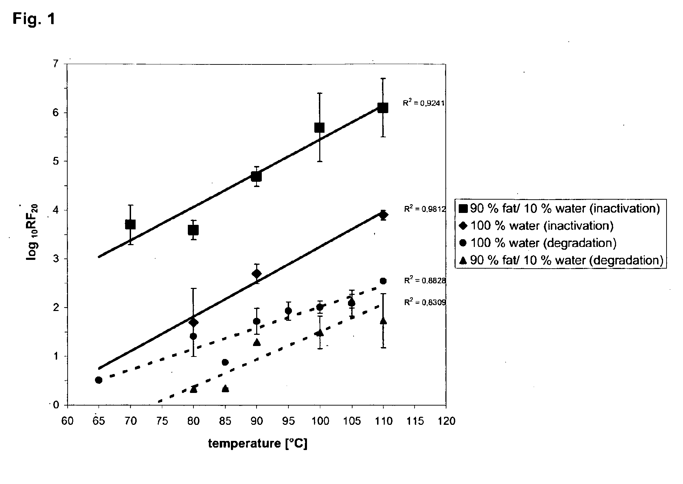

In **Figure 1** degradation and inactivation data are depicted in form of a plot of log₁₀RF₂₀ as function of temperature for heat treatment in 90 % fat / 10 % water compared to 100 % water. The quantitative evaluation was performed as described in detail by Appel *et al.* (2001) resulting in logarithmic reduction factors for a residence time of 20 min. at different temperatures (log₁₀ RF₂₀) i.e. the ratios between the amount of PrP or prion infectivity, respectively, before and after heat treatment. The solid lines in the figures represent mean values of all data points.

As shown in Figure 1, heat treatment of prion rods in different fat/water mixtures at rising temperatures yielded increasing degradation and inactivation factors. Under all conditions examined, a roughly linear temperature dependence of the reduction efficiency was measured. Comparing degradation experiments with and without tallow it became evident that the presence of fat increases the backbone intactness of PrP27-30 by one order of magnitude. In contrast, the presence of fat over the whole temperature range analysed substantially supports the prion inactivation. The inactivation-enhancing effect of fat remained constant with rising temperature. Comparing degradation data and inactivation data it became obvious, that under all conditions tested the inactivation of prion infectivity is always achieved much more efficiently than the degradation of PrP27-30 occurs.

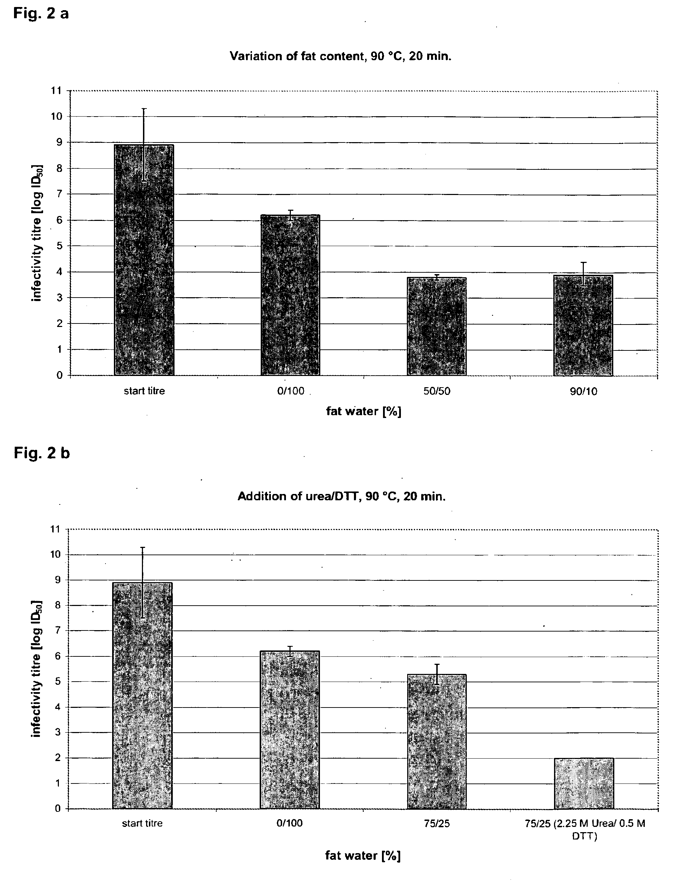

In **Figure 2** remaining infectivity titres after heat treatment at 90 °C for 20 min. are depicted as function of the relative fat content.

As can be seen in Figure 2, when comparing inactivation data of mixtures containing different amounts of fat, i.e. 50 %, 75 %, and 90 %, no substantial differences were detected. Only the presence of 2.25 M urea and 0.5 M dithiothreitol (DTT) significantly increased the prion inactivation.

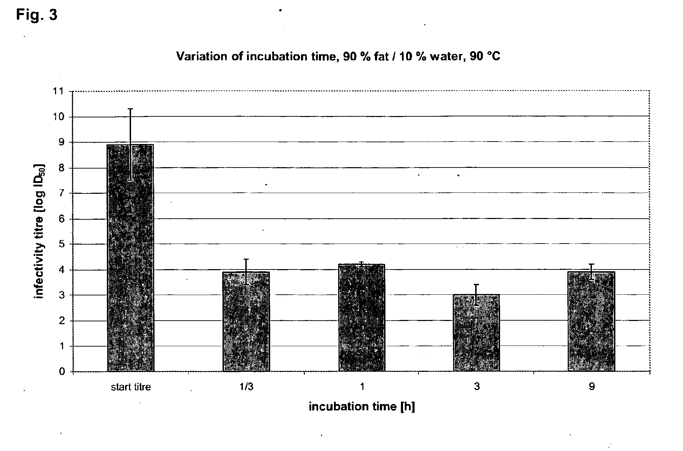

In **Figure 3** remaining infectivity titres after heat treatment in 90 % fat / 10 % water at 90 °C are depicted as function of the incubation time.

The prion inactivation achieved after incubation for 20 min., 1 h, 3 h and 9 h did not differ significantly suggesting that after 20 min. the reaction time is of minor importance for the decontamination rate emphasising the decisive effect of the reaction temperature.

It should also be noted, that physical and chemical treatment of prions appears to extend incubation periods beyond the end of the dose response curve of untreated agent compromising the estimation of infectivity titres on the basis of incubation times (Taylor and Fernie, 1996; Taylor, 1986; Dickinson and Fraser, 1969; Mould and Dawson, 1970; Lax *et al.,* 1983). According to Prusiner *et al.* (1982) and Lax *et al.* (1983) the treatment does not reduce the infectivity titre but can lengthen the incubation period by about 10 days equivalent to a discrepancy of one log ID₅₀. An endpoint dilution titration of a specimen subjected to 90 % fat and 10 % water at 90 °C for 20 min. gave a titre of 4.2 log ID₅₀/ml. An incubation time interval assay of identical conditions resulted in the same infectivity titre of 3.9 log ID₅₀/ml **(Table 1)** demonstrating that infectivity titres can be calculated on the basis of incubation times, at least under the conditions analysed. Although the degree to which infectivity titres calculated by endpoint dilution titration compared to incubation time titration appear to vary according to treatment, in this study only a limited spectrum of mild conditions was analysed. Consequently, all infectivity titres presented above are accurate.

### References:

Appel, T.R. et al. (2001, I): "Safety of oleochemical products derived from beef tallow or bone fat regarding prions", Eur. J. Lipid Sci. Technol. 103, 713-721.
Appel, T.R. et al. (2001, II): "Heat stability of prion rods and recombinant prion protein in water, lipid and lipid-water mixtures", Journal of General Virology 82, 465-473.
Dickinson, A. G., Fraser, H. (1969): "Modification of the pathogenesis of scrapie in mice by treatment of the agent", Nature 222(196):892-3.
Diringer, H., Beekes, M., Ozel, M., Simon, D., Queck, I., Cardone, F., Pocchiari, M., Ironside, J.W. (1997): "Highly infectious purified preparations of disease-specific amyloid of transmissible spongiform encephalopathies are not devoid of nucleic acids of viral size", Intervirology 40(4), 238-46".
H6rnlimann B, Leutwiler A, Oberthur RC, Widmer HR. (2001): "Die chemische Desinfektion und Inaktivierung von Prionen". Prionen & Prionerkrankungen; Berlin, deGruyter. :381-388.
Laemmli, U. K. (1970): "Cleavage of structural proteins during the assembly of the head of bacteriophage T4", Nature 227, 680-5.
Lax, A. J., Millson, G. C., Manning, E. J. (1983): "Can scrapie titres be calculated accurately from incubation periods?", J Gen Virol.; 64(4):971-3.
Mould, D. L., Dawson, A. M. (1970): "The response in mice to heat treated scrapie agent", J Comp Pathol. 80(4), 595-600.
Prusiner, S. B., McKinley, M. P., Bowman, K. A., Bolton, D. C., Bendheim, P. E., Groth, F. D., Glenner, G. G (1983): "Scrapie ,prions aggregate to form amyloid-like birefringent rods", Cell 35, 349-58.
Prusiner, S. B., Cochran, S. P., Groth, D. F., Downey, D. E., Bowman, K. A., H. Martinez, M. (1982): "Measurement of the scrapie agent using an incubation time interval assay", Ann Neurol. 11(4):353-8.
Prusiner, S.B. et al. (1981): "Scrapie agent contains a lipophilic protein", Proceedings of the National Academy of Sciences USA 78, 6675-6679.
Taylor, D. M. (1986): "Decontamination of Creutzfeldt-Jakob disease agent", Ann Neurol. 20(6):749-50.
Taylor, D. M., Fernie, K. (1996): "Exposure to autoclaving or sodium hydroxide extends the dose-response curve of the 263K strain of scrapie agent in hamsters", J Gen Virol. 77 (4):811-3.
Taylor, D.M. (2004): "Resistance of transmissible spongiform encephalopathy agents to decontamination"; Contrib. Microbiology 11; 136-45.
Wessel, D., Flügge, U. I. (1984): "A method for the quantitative recovery of protein in dilute solution in the presence of detergent and lipids", Anal Biochem. 138, 141-3.

## Claims

1. A process for the inactivation of prions in a sample, which comprises prion proteins or is suspected of comprising prion proteins, the process comprising
a heat treatment in the presence of a lipophilic substance comprising at least 8 carbon atoms
wherein the heat treatment is performed at a temperature below 100 °C at which the lipophilic substance is in a liquid state and
under conditions wherein degradation of less than 90% of the prion protein occurs.

2. The process of claim 1, wherein the lipophilic substance is selected from the group consisting of fats, fatty acids, cholesterol, oils, fatty acid derivatives, tallow and mixtures thereof.

3. The process of claim 1 or 2, wherein the conditions are selected such that degradation of less than 99 % in particular 95 % of the prion proteins occurs.

4. The process of any of claims 1 to 3, wherein the pH is above 4 and below 10.

5. The process of any of claims 1 to 4, wherein the temperature is below 95° C.

6. The process of any of claims 1 to 5, wherein the temperature is below 90° C.

7. The process of any of claims 1 to 6, wherein the heat treatment is performed in the presence of water.

8. The process of claim 7, wherein the heat treatment is performed in the presence of 5-90 % (vol/vol) water.

9. The process of any of claims 1 to 8, wherein the heat treatment is applied for a time between 2 and 60 min.

10. The process of any of claims 1 to 9, wherein the lipophilic substance is removed with an organic solvent.

11. The process of any of claims 1 to 10, wherein the sample is at least in part derived from an animal or human.

12. The process of any of claims 1 to 11, wherein the sample is a liquid, device, instrument or part of a building.

13. The process of any of claims 1 to 12, wherein the device or instrument is a device or instrument in the pharmaceutical, cosmetic or food industry or a medical device or instrument.

14. The process of any of claims 1 to 13, wherein the heat treatment its performed in the presence of at least one additive selected from the group consisting of chaotropic salts, oxidizing agents, reducing agents, detergents and aldehydes.

15. The process of any of claims 1 to 14, wherein the heat treatment is performed in the presence of at least one substance selected from the group consisting of acetone, beta- propiolactone, chlorine dioxide, diethylether, ethylene oxide, heptane, hexane, hydrogen peroxide, iodine, iodide, iodophores, potassium permanganate, sodium-dichloro-isocyanurate (NADCC), sodium periodate, peracetic acid, perchlorethylene, petrolium ether and/or benzene.

16. The process of claim 14 or 15, wherein the chaotropic salt is selected from the group consisting of urea, guanidinium hydrochloride and guanidinium isothiocyanate.

17. The process of claim 14 to 16, wherein the reducing agent is selected from the group consisting of β-mercaptoethanol and dithiothreitol.

18. A process for the inactivation of prions in a sample, which comprises prion proteins or is suspected of comprising prion proteins, the process comprising a heat treatment in the presence of a lipophilic substance comprising at least 8 carbon atoms, and further a chaotropic salt and a reducing agent, wherein the heat treatment is performed at a temperature at which the lipophilic substance is in a liquid state.

19. The process of claim 18, wherein the chaotropic salt is selected from the group consisting of urea, guanidinium hydrochloride and guanidinium isothiocyanate.

20. The process of claim 18 or 19, wherein the reducing agent is selected from the group consisting of β-mercaptoethanol and dithiothreitol.

## Patentansprüche

1. Verfahren zur Inaktivierung von Prionen in einer Probe, die Prionproteine umfasst oder von der man annimmt, dass sie Prionproteine umfassen könnte, wobei das Verfahren Folgendes umfasst:
eine Wärmebehandlung in Gegenwart einer lipophilen Substanz, die wenigstens 8 Kohlenstoffatome umfasst;
wobei die Wärmebehandlung bei einer Temperatur unter 100 °C, bei der die lipophile Substanz in einem flüssigen Zustand vorliegt, durchgeführt wird; und zwar
unter Bedingungen, bei denen ein Abbau von weniger als 90% des Prionenproteins erfolgt.

2. Verfahren gemäß Anspruch 1, wobei die lipophile Substanz aus der Gruppe ausgewählt ist, die aus Fetten, Fettsäuren, Cholesterin, Ölen, Fettsäurederivaten, Talg und Gemischen davon besteht.

3. Verfahren gemäß Anspruch 1 oder 2, wobei die Bedingungen so ausgewählt sind, dass ein Abbau von weniger als 99%, insbesondere 95%, des Prionenproteins erfolgt.

4. Verfahren gemäß einem der Ansprüche 1 bis 3, wobei der pH-Wert über 4 und unter 10 liegt.

5. Verfahren gemäß einem der Ansprüche 1 bis 4, wobei die Temperatur unter 95 °C liegt.

6. Verfahren gemäß einem der Ansprüche 1 bis 5, wobei die Temperatur unter 90 °C liegt.

7. Verfahren gemäß einem der Ansprüche 1 bis 6, wobei die Wärmebehandlung in Gegenwart von Wasser durchgeführt wird.

8. Verfahren gemäß Anspruch 7, wobei die Wärmebehandlung in Gegenwart von 5-90% (v/v) Wasser durchgeführt wird.

9. Verfahren gemäß einem der Ansprüche 1 bis 8, wobei die Wärmebehandlung während einer Zeit zwischen 2 und 60 min angewendet wird.

10. Verfahren gemäß einem der Ansprüche 1 bis 9, wobei die lipophile Substanz mit einem organischen Lösungsmittel entfernt wird.

11. Verfahren gemäß einem der Ansprüche 1 bis 10, wobei die Probe wenigstens zum Teil von einem Tier oder einem Menschen stammt.

12. Verfahren gemäß einem der Ansprüche 1 bis 11, wobei die Probe eine Flüssigkeit, eine Vorrichtung, ein Instrument oder ein Teil eines Gebäudes ist.

13. Verfahren gemäß einem der Ansprüche 1 bis 12, wobei es sich bei der Vorrichtung oder dem Instrument um eine Vorrichtung bzw. ein Instrument in der pharmazeutischen, kosmetischen oder Nahrungsmittelindustrie oder um eine medizinische Vorrichtung oder ein medizinisches Instrument handelt.

14. Verfahren gemäß einem der Ansprüche 1 bis 13, wobei die Wärmebehandlung in Gegenwart wenigstens eines Additivs durchgeführt wird, das aus der Gruppe ausgewählt ist, die aus chaotropen Salzen, Oxidationsmitteln, Reduktionsmitteln, Detergentien und Aldehyden besteht.

15. Verfahren gemäß einem der Ansprüche 1 bis 14, wobei die Wärmebehandlung in Gegenwart wenigstens einer Substanz durchgeführt wird, die aus der Gruppe ausgewählt ist, die aus Aceton, beta-Propiolacton, Chlordioxid, Diethylether, Ethylenoxid, Heptan, Hexan, Wasserstoffperoxid, Iod, Iodid, Iodophoren, Kaliumpermanganat, Natriumdichlorisocyanurat (NADCC), Natriumperiodat, Peressigsäure, Perchlorethylen, Petrolether und/oder Benzol besteht.

16. Verfahren gemäß Anspruch 14 oder 15, wobei das chaotrope Salz aus der Gruppe ausgewählt ist, die aus Harnstoff, Guanidiniumchlorid (Guanidin-Hydrochlorid) und Guanidiniumisothiocyanat besteht.

17. Verfahren gemäß Anspruch 14 bis 16, wobei das Reduktionsmittel aus der Gruppe ausgewählt ist, die aus β-Mercaptoethanol und Dithiothreit besteht.

18. Verfahren zur Inaktivierung von Prionen in einer Probe, die Prionproteine umfasst oder von der man annimmt, dass sie Prionproteine umfassen könnte, wobei das Verfahren eine Wärmebehandlung in Gegenwart einer lipophilen Substanz, die wenigstens 8 Kohlenstoffatome umfasst, und weiterhin eines chaotropen Salzes und eines Reduktionsmittels umfasst, wobei die Wärmebehandlung bei einer Temperatur durchgeführt wird, bei der die lipophile Substanz in einem flüssigen Zustand vorliegt.

19. Verfahren gemäß Anspruch 18, wobei das chaotrope Salz aus der Gruppe ausgewählt ist, die aus Harnstoff, Guanidiniumchlorid (Guanidin-Hydrochlorid) und Guanidiniumisothiocyanat besteht.

20. Verfahren gemäß Anspruch 18 oder 19, wobei das Reduktionsmittel aus der Gruppe ausgewählt ist, die aus β-Mercaptoethanol und Dithiothreit besteht.

## Revendications

1. Procédé d'inactivation des prions dans un échantillon qui contient des protéines de prions ou qui est présumé contenir des protéines de prions, le procédé comprenant :
un traitement thermique en présence d'une substance lipophile renfermant au moins 8 atomes de carbone ;
le traitement thermique étant effectué à une température inférieure à 100 °C à laquelle la substance lipophile est à l'état liquide, et
dans des conditions où se produit une dégradation du prion inférieure à 90 %.

2. Procédé selon la revendication 1, dans lequel la substance lipophile est choisie dans le groupe consistant en matières grasses, acides gras, le cholestérol, des huiles, des dérivés d'acides gras, du suif et des mélanges de ceux-ci.

3. Procédé selon la revendication 1 ou 2, dans lequel les conditions sont choisies de manière qu'une dégradation de moins de 99 %, notamment 95 %, des protéines de prions ait lieu.

4. Procédé selon l'une quelconque des revendications 1 à 3, dans lequel la valeur de pH est supérieure à 4 et inférieure à 10.

5. Procédé selon l'une quelconque des revendications 1 à 4, dans lequel la température est inférieure à 95 °C.

6. Procédé selon l'une quelconque des revendications 1 à 5, dans lequel la température est inférieure à 90 °C.

7. Procédé selon l'une quelconque des revendications 1 à 6, dans lequel le traitement à chaud est réalisé en présence d'eau.

8. Procédé selon la revendication 7, dans lequel le traitement à chaud est réalisé en présence de 5 à 90 % (vol/vol) d'eau.

9. Procédé selon l'une quelconque des revendications 1 à 8, dans lequel le traitement à chaud est appliqué pendant une durée comprise entre 2 et 60 min.

10. Procédé selon l'une quelconque des revendications 1 à 9, dans lequel la substance lipophile est éliminée avec un solvant organique.

11. Procédé selon l'une quelconque des revendications 1 à 10, dans lequel l'échantillon provient au moins partiellement d'un animal ou d'un humain.

12. Procédé selon l'une quelconque des revendications 1 à 11, dans lequel l'échantillon est un liquide, un dispositif, un instrument ou une partie d'un bâtiment.

13. Procédé selon l'une quelconque des revendications 1 à 12, dans lequel le dispositif ou l'instrument est un dispositif ou un instrument dans l'industrie pharmaceutique, cosmétique ou alimentaire ou un dispositif ou instrument médical.

14. Procédé selon l'une quelconque des revendications 1 à 13, dans lequel le traitement à chaud est réalisé en présence d'au moins un additif choisi dans le groupe consistant en sels chaotropiques, agents oxydants, agents réducteurs, détergents et aldéhydes.

15. Procédé selon l'une quelconque des revendications 1 à 14, dans lequel le traitement à chaud est réalisé en présence d'au moins une substance choisie dans le groupe consistant en acétone, bêta-propiolactone, dioxyde de chlore, éther diéthylique, oxyde d'éthylène, heptane, hexane, peroxyde d'hydrogène, iode, iodure, iodophores, permanganate de potassium, dichloroisocyanurate de sodium (NADCC), periodate de sodium, acide peracétique, perchloroéthylène, éther de pétrole et/ou benzène.

16. Procédé selon la revendication 14 ou 15, dans lequel le sel chaotropique est choisi dans le groupe consistant en urée, hydrochlorure de guanidine (chlorure de guanidinium) et isothiocyanate de guanidinium.

17. Procédé selon la revendication 14 à 16, dans lequel l'agent réducteur est choisi dans le groupe consistant en β-mercaptoéthanol et dithiothréitol.

18. Procédé d'inactivation des prions dans un échantillon qui contient des protéines de prions ou qui est présumé contenir des protéines de prions, le procédé comprenant un traitement thermique en présence d'une substance lipophile renfermant au moins 8 atomes de carbone et en outre d'un sel chaotropique et d'un agent réducteur, dans lequel le traitement thermique est effectué à une température à laquelle la substance lipophile est à l'état liquide.

19. Procédé selon la revendication 18, dans lequel le sel chaotropique est choisi dans le groupe consistant en urée, hydrochlorure de guanidine (chlorure de guanidinium) et isothiocyanate de guanidinium.

20. Procédé selon la revendication 18 ou 19, dans lequel l'agent réducteur est choisi dans le groupe consistant en β-mercaptoéthanol et dithiothréitol.
